Europäisches Patentamt

(19) European Patent Office

Office européen des brevets

(11) **EP 0 750 493 B1**

## (12) FASCICULE DE BREVET EUROPEEN

(45) Date de publication et mention
de la délivrance du brevet:
**14.10.1998 Bulletin 1998/42**

(21) Numéro de dépôt: **95913216.8**

(22) Date de dépôt: **17.03.1995**

(51) Int. Cl.⁶: **A61K 9/00**, A61K 9/20

(86) Numéro de dépôt international:
**PCT/FR95/00332**

(87) Numéro de publication internationale:
**WO 95/24890 (21.09.1995 Gazette 1995/40)**

(54) **PROCEDE DE FABRICATION DE COMPRIMES A CROQUER A BASE DE TROXERUTINE, DE CARBONATE DE CALCIUM, DE PHOSPHATE DE CALCIUM, D'ASPARTATE D'ARGININE, DE GLUTAMATE D'ARGININE, D'AMOXICILLINE, DE COMBINAISON DE CARBONATE DE CALCIUM ET DE CARBONATE DE MAGNESIUM ET DE COMBINAISON D'HYDROXYDE D'ALUMINIUM ET D'H**

VERFAHREN ZUR HERSTELLUNG VON KAUTABLETTEN AUS TROXERUTIN, KALZIUMCARBONAT KALZIUMPHOSPHAT, ARGININASPARTAT, ARGININGLUTAMAT, AMOXICILLIN, EINE KOMBINATION AUS KALZIUMCARBONAT UND MAGNESIUMCARBONAT UND EINE KOMBINATION AUS ALUMINIUMHYDROXYD UND MAGNESIUMHYDROXYD

METHOD FOR MAKING CHEWABLE TABLETS BASED ON TROXERUTINE, CALCIUM CARBONATE, CALCIUM PHOSPHATE, ARGININE ASPARTATE, ARGININE GLUTAMATE, AMOXICILLIN, A COMBINATION OF CALCIUM CARBONATE AND MAGNESIUM CARBONATE, AND A COMBINATION OF ALUMINIUM HYDROXIDE AND MAGNESIUM HYDROXIDE

(84) Etats contractants désignés:
**DE FR GB IT NL**

(30) Priorité: **17.03.1994 FR 9403142**

(43) Date de publication de la demande:
**02.01.1997 Bulletin 1997/01**

(73) Titulaire: **HI PHARMTECH**
**28170 Chateauneuf-en-Thymerais (FR)**

(72) Inventeurs:
• **MARTANI, Rosa**
**06150 Cannes (FR)**
• **DEMICHELIS, Alain Gilles**
**06130 Grasse (FR)**

• **BARBERO née WOILTOCK, Maryvonne**
**06565 Valbonne (FR)**
• **LE VU, Dominique**
**06500 Menton (FR)**
• **LECOINTRE, Bertrand**
**06100 Nice (FR)**
• **RABOT, Patricia**
**06250 Mougins (FR)**

(74) Mandataire: **Koch, Gustave et al**
**Cabinet PLASSERAUD**
**84, rue d'Amsterdam**
**75440 Paris Cédex 09 (FR)**

(56) Documents cités:
**US-A- 4 242 323**

## Description

L'invention a pour objet un procédé de fabrication de comprimés à croquer dont la substance active est choisie dans le groupe comprenant la troxérutine, le carbonate de calcium, le phosphate de calcium, l'aspartate d'arginine, le glutamate d'arginine, l'amoxicilline, la combinaison de carbonate de calcium et de carbonate de magnésium et la combinaison d'hydroxyde d'aluminium et d'hydroxyde de magnésium.

L'invention a également pour objet des comprimés à croquer dont la substance active est choisie dans le groupe comprenant la troxérutine, le carbonate de calcium, le phosphate de calcium, l'aspartate d'arginine, le glutamate d'arginine, l'amoxicilline, la combinaison de carbonate de calcium et de carbonate de magnésium et la combinaison d'hydroxyde d'aluminium et d'hydroxyde de magnésium, et qui sont susceptibles d'être obtenus par ce procédé.

Il est connu que les substances actives venant d'être énumérées présentent un goût et/ou une odeur désagréables.

Cette particularité est gênante étant donné qu'elle conduit à des réticences du point de vue du volume et de l'absorption des comprimés à base de ces substances, notamment de la part des enfants et des personnes âgées.

Il a déjà été proposé de remédier à cet état de choses en faisant comporter aux comprimés à base des substances actives en question un enrobage qui peut être à base de sucre, de matériaux cireux, de matériaux à haut poids moléculaire, de lipides ou de mélange de lipides.

Il a également été proposé de faire comporter aux comprimés du genre en question des arômes, des édulcorants ou encore des huiles essentielles, notamment de Citrus.

Aucune de ces solutions n'a donné entièrement satisfaction.

L'invention a donc pour but, surtout, de remédier aux inconvénients de l'art antérieur et de fournir un procédé propre à permettre la fabrication de comprimés à croquer à base des substances actives susmentionnées donnant entièrement satisfaction.

Or, la Société Demanderesse a eu le mérite de trouver à l'issue de recherches approfondies que ce but pouvait être atteint dès lors qu'il est fait recours à de la poudre de cacao à titre d'agent de masquage du goût et/ou des odeurs désagréables générées par les substances actives en question.

Le mérite de la Société Demanderesse est d'autant plus grand, qu'elle a trouvé que la mise en oeuvre de la poudre de cacao --dont on connaissait, par le document US-A-4 242 323, l'utilisation dans des compositions orales ayant des propriétés inhibitrices de la plaque dentaire--en tant qu'agent de masquage du mauvais goût et/ou des odeurs désagréables des substances actives en question, permettait au surplus de supprimer les agents de compression classiques, la poudre de cacao s'étant montrée de façon surprenante et inattendue apte à jouer le rôle d'agent de compression, dès lors qu'elle est mise en oeuvre en une proportion suffisante.

En conséquence, le procédé de fabrication conforme à l'invention de comprimés à croquer à base de carbonate de calcium, de phosphate de calcium, de troxérutine, d'aspartate d'arginine, de glutamate d'arginine, d'amoxicilline, de combinaison de carbonate de calcium et de carbonate de magnésium et de combinaison d'hydroxyde d'aluminium et d'hydroxyde de magnésium, est caractérisé par le fait qu'avant l'étape de compression proprement dite, on fait comporter au mélange des substances entrant dans la constitution du comprimé une proportion suffisante de poudre de cacao, cette proportion étant d'environ 1% à environ 50% en poids et de préférence de 14 à 30% en poids par rapport à la masse totale du comprimé.

Selon un mode de réalisation avantageux du susdit procédé, la poudre de cacao mise en oeuvre présente une granulométrie de 25 à 75 micromètres.

On rappelle tout d'abord que, comme connu, le procédé de fabrication d'un comprimé à croquer comprend les étapes suivantes :

- mélange de la poudre de cacao avec la substance active et les excipients entrant dans la constitution du comprimé à préparer,
- mouillage, notamment avec de l'eau ou de l'alcool jusqu'à l'obtention d'une masse humide, dont la teneur en matières sèches est de 80% à 95%,
- granulation de la masse humide,
- séchage des granulés obtenus,
- calibrage des granulés,
- mélange avec, éventuellement, d'autres excipients (exemples : édulcorants, aromatisants et lubrifiants),
- compression du mélange final.

L'invention pourra être encore mieux comprise à l'aide des exemples non limitatifs qui suivent et qui constituent des modes de réalisation avantageux du procédé conforme à l'invention. L'invention est définie par les revendications.

### EXEMPLE 1

Préparation d'un comprimé à croquer à base de carbonate de calcium

Dans un premier temps, on réalise le mélange de 125 parties en poids de carbonate de calcium, de 25 parties en poids de poudre de cacao, de 20 parties en poids de sorbitol et 3 parties en poids de polyvidone.

Le mélange ainsi réalisé est mouillé avec 28 parties d'eau purifiée, ce qui permet d'obtenir une masse humide ayant une teneur en matières sèches de 85% à 87%.

La masse humide ainsi obtenue est granulée, par exemple sur un granulateur oscillant équipé d'une grille de vide de maille de 1,6 mm.

Les granules ainsi obtenus sont séchés, notamment à l'intérieur d'une étuve, à une température d'environ de vide de maille de 1,6 mm.

Les granules ainsi obtenus sont séchés, notamment à l'intérieur d'une étuve, à une température d'environ 50°C jusqu'à l'obtention d'une humidité relative de 2%.

Les granules secs ainsi obtenus sont calibrés sur une grille de vide de maille de 1 mm puis mélangés avec une quantité correspondante à 0,2 partie en poids d'aspartam, 1,4 parties en poids d'arôme noisette, 1 partie en poids de talc et 0,4 partie en poids de stéarate de magnésium.

Le mélange obtenu est homogénéisé.

A partir de ce mélange homogénéisé, on prépare des comprimés d'un poids unitaire de 1760 mg et d'une dureté de 8 à 10 kg.

Les comprimés sont ensuite dragéifiés selon le procédé conventionnel. L'enrobage, d'un poids d'environ 490 mg pour un comprimé, comporte de la gomme laque, du maltitol, de la gélatine, de la vanilline, du talc, du dioxyde de titane et de la cire d'abeille.

La composition des dragées est:

| Carbonate de calcium | 1250 mg |
|---|---|
| Poudre de cacao | 250 mg |
| Sorbitol | 200 mg |
| Polyvidone | 30 mg |
| Aspartam | 2 mg |
| Arôme noisette | 14 mg |
| Talc | 10 mg |
| Stéarate de magnésium | 4 mg |
| | 1760 mg |

## EXEMPLE 2

### Préparation d'un comprimé à croquer à base de phosphate de calcium

Dans un premier temps, on réalise le mélange de 160 parties en poids de phosphate de calcium, de 42 parties en poids de poudre de cacao, de 15 parties en poids de sorbitol et 5 parties en poids de polyvidone.

Le mélange ainsi réalisé est mouillé avec 30 parties d'eau purifiée, ce qui permet d'obtenir une masse humide ayant une teneur en matières sèches de 88% à 90%.

La masse humide ainsi obtenue est granulée, par exemple sur un granulateur oscillant équipé d'une grille de vide de maille de 1,6 mm.

Les granules ainsi obtenus sont séchés, notamment à l'intérieur d'une étuve, à une température d'environ 50°C jusqu'à l'obtention d'une humidité relative de 2%.

Les granules secs ainsi obtenus sont calibrés sur une grille de vide de maille de 1 mm puis mélangés avec une quantité correspondante à 1 partie en poids d'aspartam, 4 parties en poids d'arôme orange, 2 parties en poids de talc et 1 partie en poids de stéarate de magnésium.

Le mélange obtenu est homogénéisé.

A partir de ce mélange homogénéisé, on prépare des comprimés d'un poids unitaire de 2300 mg et d'une dureté de 8 à 10 kg.

La composition des comprimés est:

| Phosphate neutre de calcium | 1600 mg |
|---|---|
| Poudre de cacao | 420 mg |
| Sorbitol | 150 mg |
| Polyvidone | 50 mg |
| Aspartam | 10 mg |
| Arôme orange | 40 mg |
| Talc | 20 mg |
| Stéarate de magnésium | 10 mg |
| | 2300 mg |

## EXEMPLE 3

### Préparation d'un comprimé à croquer à base de troxérutine

Dans un premier temps, on réalise le mélange de 100 parties en poids de troxérutine, de 40 parties en poids de poudre de cacao, de 20 parties en poids de mannitol.

Le mélange ainsi réalisé est mouillé avec 38 parties d'éthanol à 80 volumes, ce qui permet d'obtenir une masse humide ayant une teneur en matières sèches de 80% à 82%.

La masse humide ainsi obtenue est granulée, par exemple sur un granulateur oscillant équipé d'une grille de vide de maille de 1,6 mm.

Les granules ainsi obtenus sont séchés, notamment à l'intérieur d'une étuve, à une température d'environ 50°C jusqu'à l'obtention d'une humidité relative de 2%.

Les granules secs ainsi obtenus sont calibrés sur une grille de vide de maille de 1 mm puis mélangés avec une quantité correspondante à 0,6 partie en poids d'aspartam, 3 parties en poids d'arôme orange, 1,6 parties en poids de talc et 0,8 partie en poids de stéarate de magnésium.

Le mélange obtenu est homogénéisé.

A partir de ce mélange homogénéisé, on prépare des comprimés d'un poids unitaire de 1660 mg et d'une dureté de 8 à 10 kg.

La composition des comprimés est:

| Troxérutine | 1000 mg |
|---|---|
| Poudre de cacao | 400 mg |
| Mannitol | 200 mg |
| Aspartam | 6 mg |
| Arôme orange | 30 mg |
| Talc | 16 mg |
| Stéarate de magnésium | 8 mg |
| | 1660 mg |

### EXEMPLE 4

Préparation d'un comprimé à croquer à base d'amoxicilline

On réalise le mélange de 100 parties en poids d'amoxicilline, de 45,6 parties en poids de poudre de cacao, de 4 parties en poids d'édulcorant, notamment d'aspartam, de 0,4 partie de silice colloïdale, 1 partie de talc, 1 partie de stéarate de magnésium et 8 parties d'arôme mandarine ou antillais.

Le mélange ainsi obtenu est homogénéisé.

A partir de ce mélange homogénéisé, on prépare des comprimés d'un poids unitaire de 1600 mg et d'une dureté de 6 à 8 kg.

La composition des comprimés est:

| Amoxicilline trihydrate compactée | 1000 mg |
|---|---|
| Poudre de cacao | 456 mg |
| Aspartam | 40 mg |
| Silice colloïdale | 4 mg |
| Talc | 10 mg |
| Stéarate de magnésium | 10 mg |
| Arôme mandarine ou antillais | 80 mg |
| | 1600 mg |

### EXEMPLE 5

Préparation d'un comprimé à croquer à base d'aspartate d'arginine

On réalise le mélange de 100 parties en poids d'aspartate d'arginine, de 43 parties en poids de poudre de cacao, de 1,5 parties en poids de polyvidone présentant une constante de viscosité K égale à 30.

Le mélange ainsi réalisé est mouillé avec 10 parties d'eau purifiée, ce qui permet d'obtenir une masse humide ayant une teneur en matières sèches de 92% à 94%.

La masse humide ainsi obtenue est granulée, par exemple sur un granulateur oscillant équipé d'une grille de vide de maille de 1,6 mm.

Les granules ainsi obtenus sont séchés, notamment à l'intérieur d'une étuve, à une température d'environ 50°C jusqu'à l'obtention d'une humidité relative de 1%.

Les granules secs ainsi obtenus sont calibrés sur une grille de vide de maille de 1 mm puis mélangés avec une quantité correspondante à 2 parties en poids d'aspartam, 1 partie en poids de talc et 1 partie en poids de stéarate de magnésium et 5 parties en poids d'arôme café.

Le mélange obtenu est homogénéisé.

A partir de ce mélange homogénéisé, on prépare des comprimés d'un poids unitaire de 1535 mg et d'une dureté de 6 à 8 kg.

La composition des comprimés est:

| Aspartate d'arginine | 1000 mg |
|---|---|
| Poudre de cacao | 430 mg |
| Polyvidone (K = 30) | 15 mg |
| Aspartam | 20 mg |
| Talc | 10 mg |
| Stéarate de magnésium | 10 mg |
| Arôme café | 50 mg |
| | 1535 mg |

### EXEMPLE 6

Préparation d'un comprimé à croquer à base de carbonate de calcium et de carbonate de magnésium

On réalise le mélange de 46 parties en poids de carbonate de calcium, de 14 parties en poids de carbonate de magnésium, de 25 parties en poids de poudre de cacao, de 20 parties en poids de sorbitol et de 3 parties en poids de polyvidone.

Le mélange ainsi réalisé est mouillé avec 25 parties d'eau purifiée, ce qui permet d'obtenir une masse humide ayant une teneur en matières sèches de 80% à 82%.

La masse humide ainsi obtenue est granulée, par exemple sur un granulateur oscillant équipé d'une grille de vide de maille de 1,6 mm.

Les granules ainsi obtenus sont séchés, notamment à l'intérieur d'une étuve, à une température d'environ 50°C jusqu'à l'obtention d'une humidité relative de 2%.

Les granules secs ainsi obtenus sont calibrés sur une grille de vide de maille de 1 mm puis mélangés avec une quantité correspondante à 0,2 partie en poids d'aspartam, 1,4 partie en poids d'arôme noisette, 1 partie en poids de talc et 0,4 partie en poids de stéarate de magnésium.

Le mélange obtenu est homogénéisé.

A partir de ce mélange homogénéisé, on prépare des comprimés d'un poids unitaire de 1110 mg et d'une dureté de 8 à 10 kg.

Les comprimés sont ensuite dragéifiés en procédant de manière conventionnelle. L'enrobage, d'un poids d'environ 490 mg pour un comprimé, comporte de la gomme laque, du maltitol, de la gélatine, de la vanilline, du talc, du dioxyde de titane et de la cire d'abeille.

La composition des comprimés est:

| Carbonate de calcium | 460 mg |
| Carbonate de magnésium | 140 mg |
| Poudre de cacao | 250 mg |
| Sorbitol | 200 mg |
| Polyvidone | 30 mg |
| Aspartam | 2 mg |
| Arôme noisette | 14 mg |
| Talc | 10 mg |
| Stéarate de magnésium | 4 mg |
| | 1110 mg |

### EXEMPLE 7

#### Préparation d'un comprimé à croquer à base d'hydroxyde d'aluminium et d'hydroxyde de magnésium

On réalise le mélange de 40 parties en poids d'hydroxyde d'aluminium, de 40 parties en poids d'hydroxyde de magnésium, de 45 parties en poids de poudre de cacao et de 3 parties en poids de polyvidone.

Le mélange ainsi réalisé est mouillé avec 18 parties d'eau purifiée, ce qui permet d'obtenir une masse humide ayant une teneur en matières sèches de 87% à 89%.

La masse humide ainsi obtenue est granulée, par exemple sur un granulateur oscillant équipé d'une grille de vide de maille de 1,6 mm.

Les granules ainsi obtenus sont séchés, notamment à l'intérieur d'une étuve, à une température d'environ 50°C jusqu'à l'obtention d'une humidité relative de 2%.

Les granules secs ainsi obtenus sont calibrés sur une grille de vide de maille de 1 mm puis mélangés avec une quantité correspondante à 0,2 partie en poids d'aspartam, 1,4 partie en poids d'arôme noisette, 1 partie en poids de talc et 0,4 partie en poids de stéarate de magnésium.

Le mélange obtenu est homogénéisé.

A partir de ce mélange homogénéisé, on prépare des comprimés d'un poids unitaire de 1310 mg et d'une dureté de 7 à 10 kg.

Les comprimés sont ensuite dragéifiés en procédant de manière conventionnelle. L'enrobage, d'un poids d'environ 490 mg pour un comprimé, comporte de la gomme laque, du maltitol, de la gélatine, de la vanilline, du talc, du dioxyde de titane et de la cire d'abeille.

La composition des comprimés est:

| Hydroxyde d'aluminium | 400 mg |
| Hydroxyde de magnésium | 400 mg |
| Poudre de cacao | 450 mg |
| Polyvidone | 30 mg |
| Aspartam | 2 mg |
| Arôme noisette | 14 mg |
| Talc | 10 mg |
| Stéarate de magnésium | 4 mg |
| | 1310 mg |

### Revendications

1. Utilisation, à titre d'agent de masquage du goût et d'agent de compression dans le cadre d'un procédé de préparation de comprimés à croquer dont la substance active est choisie dans le groupe comprenant la troxérutine, le carbonate de calcium, le phosphate de calcium, l'aspartate d'arginine, le glutamate d'arginine et l'amoxicilline, la combinaison de carbonate de calcium et de carbonate de magnésium et la combinaison d'hydroxyde d'aluminium et d'hydroxyde de magnésium, d'une quantité efficace de poudre de cacao.

2. Utilisation selon la revendication 1 d'une quantité de poudre de cacao comprise entre 1 et 50% en poids et de préférence, entre 14 et 30% en poids par rapport à la masse totale du comprimé.

3. Procédé de préparation de comprimés à croquer dont la substance active est choisie dans le groupe comprenant la troxérutine, le carbonate de calcium, le phosphate de calcium, l'aspartate d'arginine, le glutamate d'arginine et l'amoxicilline, la combinaison de carbonate de calcium et de carbonate de magnésium et la combinaison d'hydroxyde d'aluminium et d'hydroxyde de magnésium, caractérisé par le fait que l'on fait comporter au comprimé une quantité efficace, de préférence comprise entre 1 et 50% en poids et plus préférentiellement encore entre 14 et 30% en poids par rapport à la masse totale du comprimé, d'une poudre de cacao présentant une granulométrie de 25 à 75 micromètres et utilisée à titre d'agent de masquage du goût et de compression.

4. Comprimé à croquer à base de troxérutine, caractérisé en ce qu'il contient, à titre d'agent de compression et de masquage du goût, une quantité de poudre de cacao de 24% en poids par rapport à la masse totale des comprimés.

5. Comprimé à croquer à base de carbonate de calcium, caractérisé en ce qu'il contient, à titre d'agent de compression et de masquage du goût, une quantité de poudre de cacao de 14% en poids par rapport à la masse totale des comprimés.

6. Comprimé à croquer à base d'amoxicilline, caractérisé en ce qu'il contient, à titre d'agent de compression et de masquage du goût, une quantité de poudre de cacao de 29% en poids par rapport à la masse totale des comprimés.

7. Comprimé à croquer à base de phosphate de calcium, caractérisé en ce qu'il contient, à titre d'agent de compression et de masquage du goût, une quantité de poudre de cacao de 18% en poids par rapport à la masse totale des comprimés.

8. Comprimé à croquer à base d'aspartate d'arginine, caractérisé en ce qu'il contient, à titre d'agent de compression et de masquage du goût, une quantité de poudre de cacao de 28% en poids par rapport à la masse totale des comprimés.

9. Comprimé à croquer à base de carbonate de calcium et de carbonate de magnésium, caractérisé en ce qu'il contient, à titre d'agent de compression et de masquage du goût, une quantité de poudre de cacao de 22% en poids par rapport à la masse totale des comprimés.

10. Comprimé à croquer à base d'hydroxyde d'aluminium et d'hydroxyde de magnésium, caractérisé en ce qu'il contient, à titre d'agent de compression et de masquage du goût, une quantité de poudre de cacao de 34% en poids par rapport à la masse totale des comprimés.

**Claims**

1. Use, as taste masking agent and compressing agent within the scope of a method for preparing chewable tablets wherein the active substance is selected from the group comprising troxerutine, calcium carbonate, calcium phosphate, arginine aspartate, arginine glutamate and amoxicillin, the combination of calcium carbonate and magnesium carbonate and the combination of aluminum hydroxide and magnesium hydroxide, of an efficient proportion of cocoa powder.

2. Use according to claim 1 of a proportion of cocoa powder comprised between 1 and 50% by weight and preferably between 14 and 30% by weight with respect to the total mass of the tablet.

3. Method for preparing chewable tablets wherein the active substance is selected from the group comprising troxerutine, calcium carbonate, calcium phosphate, arginine aspartate, arginine glutamate and amoxicillin, the combination of calcium carbonate and magnesium carbonate and the combination of aluminum hydroxide and magnesium hydroxide, characterized by the fact that the tablet comprises an efficient proportion, preferably comprised between 1 and 50% by weight and still more preferably between 14 and 30% by weight with respect to the total mass of the tablet, of a cocoa powder presenting a particle size from 25 to 75 microns and used as taste masking agent and compressing agent.

4. Chewable tablet based on troxerutine, characterized by the fact that it comprises, as compressing agent and taste masking agent, a proportion of cocoa powder of 24% by weight with respect to the total mass of the tablets.

5. Chewable tablet based on calcium carbonate, characterized by the fact that it comprises, as compressing agent and taste masking agent, a proportion of cocoa powder of 14% by weight with respect to the total mass of the tablets.

6. Chewable tablet based on amoxicilline, characterized by the fact that it comprises, as compressing agent and taste masking agent, a proportion of cocoa powder of 29% by weight with respect to the total mass of the tablets.

7. Chewable tablet based on calcium phosphate, characterized by the fact that it comprises, as compressing agent and taste masking agent, a proportion of cocoa powder of 18% by weight with respect to the total mass of the tablets.

8. Chewable tablet based on arginine aspartate, characterized by the fact that it comprises, as compressing agent and taste masking agent, a proportion of cocoa powder of 28% by weight with respect to the total mass of the tablets.

9. Chewable tablet based on calcium carbonate and magnesium carbonate, characterized by the fact that it comprises, as compressing agent and taste masking agent, a proportion of cocoa powder of 22% by weight with respect to the total mass of the tablets.

10. Chewable tablet based on aluminum hydroxide and magnesium hydroxide, characterized by the fact that it comprises, as compressing agent and taste masking agent, a proportion of cocoa powder of 34% by weight with respect to the total mass of the tablets.

**Patentansprüche**

1. Verwendung einer wirksamen Menge von Kakaopulver als Geschmacksmaskierungs- und Preßhilfsmittel im Rahmen eines Verfahrens zur Herstellung von Kautabletten, deren Wirkstoff aus der Gruppe ausgewählt ist, welche Troxerutin, Calciumcarbonat, Calciumphosphat, Argininaspartat, Argininglutamat und Amoxicillin, die Kombination von Calciumcarbonat und Magnesiumcarbonat und die Kombination von Aluminiumhydroxid und Magnesiumhydroxid umfaßt.

2. Verwendung nach Anspruch 1 einer Menge von Kakaopulver, die zwischen 1 und 50 Gew.-% und bevorzugt zwischen 14 und 30 Gew.-% in Bezug auf die Gesamtmasse der Tablette beträgt.

3. Verfahren zur Herstellung von Kautabletten, deren Wirkstoff aus der Gruppe ausgewählt ist, welche Troxerutin, Calciumcarbonat, Calciumphosphat, Argininaspartat, Argininglutamat und Amoxicillin, die Kombination von Calciumcarbonat und Magnesiumcarbonat und die Kombination von Aluminiumhydroxid und Magnesiumhydroxid umfaßt, dadurch gekennzeichnet, daß der Tablette eine bevorzugt zwischen 1 und 50 Gew.-% und insbesondere bevorzugt zwischen 14 und 30 Gew.-% in Bezug auf die Gesamtmasse der Tablette betragende wirksame Menge eines als Geschmacksmaskierungs-und Preßhilfsmittel eingesetzten Kakaopulvers mit einer Korngröße von 25 bis 75 μm beigegeben wird.

4. Kautablette auf der Basis von Troxerutin, dadurch gekennzeichnet, daß sie als Preßhilfs- und Geschmacksmaskierungsmittel eine Kakaopulvermenge von 24 Gew.-% in Bezug auf die Gesamtmasse der Tabletten enthält.

5. Kautablette auf der Basis von Calciumcarbonat, dadurch gekennzeichnet, daß sie als Preßhilfs- und Geschmacksmaskierungsmittel eine Kakaopulvermenge von 14 Gew.-% in Bezug auf die Gesamtmasse der Tabletten enthält.

6. Kautablette auf der Basis von Amoxicillin, dadurch gekennzeichnet, daß sie als Preßhilfs- und Geschmacksmaskierungsmittel - eine Kakaopulvermenge von 29 Gew.-% in Bezug auf die Gesamtmasse der Tabletten enthält.

7. Kautablette auf der Basis von Calciumphosphat, dadurch gekennzeichnet, daß sie als Preßhilfs- und Geschmacksmaskierungsmittel eine Kakaopulvermenge von 18 Gew.-% in Bezug auf die Gesamtmasse der Tabletten enthält.

8. Kautablette auf der Basis von Argininaspartat, dadurch gekennzeichnet, daß sie als Preßhilfs- und Geschmacksmaskierungsmittel eine Kakaopulvermenge von 28 Gew.-% in Bezug auf die Gesamtmasse der Tabletten enthält.

9. Kautablette auf der Basis von Calciumcarbonat und Magnesiumcarbonat, dadurch gekennzeichnet, daß sie als Preßhilfs- und Geschmacksmaskierungsmittel eine Kakaopulvermenge von 22 Gew.-% in Bezug auf die Gesamtmasse der Tabletten enthält.

10. Kautablette auf der Basis von Aluminiumhydroxid und Magnesiumhydroxid, dadurch gekennzeichnet, daß sie als Preßhilfs- und Geschmacksmaskierungsmittel eine Kakaopulvermenge von 34 Gew.-% in Bezug auf die Gesamtmasse der Tabletten enthält.